(19) **Europäisches Patentamt / European Patent Office / Office européen des brevets**

(11) **EP 3 626 171 A1**

(12) **EUROPEAN PATENT APPLICATION**
published in accordance with Art. 153(4) EPC

(43) Date of publication:
**25.03.2020 Bulletin 2020/13**

(21) Application number: **17909872.8**

(22) Date of filing: **19.05.2017**

(51) Int Cl.:
*A61B 5/1468* (2006.01)      *G01N 33/49* (2006.01)
*G01N 33/487* (2006.01)      *A61B 5/145* (2006.01)
*A61B 5/00* (2006.01)

(86) International application number:
**PCT/KR2017/005252**

(87) International publication number:
**WO 2018/212384 (22.11.2018 Gazette 2018/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.05.2017 KR 20170061460**

(71) Applicant: **Micobiomed Co., Ltd.**
**Gyeonggi-do 13449 (KR)**

(72) Inventors:
• **JEONG, Jong Min**
  **Anseong-si**
  **Gyeonggi-do 17546 (KR)**
• **HAN, Jin Keun**
  **Wonju-si**
  **Gangwon-do 26491 (KR)**
• **KIM, Yong Min**
  **Anseong-si**
  **Gyeonggi-do 17546 (KR)**
• **LIM, Sun Hee**
  **Seoul 06139 (KR)**
• **LEE, Ji Yoon**
  **Anseong-si**
  **Gyeonggi-do 17546 (KR)**
• **AN, Byeong Chan**
  **Gunsan-si**
  **Jeollabuk-do 54153 (KR)**
• **SONG, Won Seok**
  **Anseong-si**
  **Gyeonggi-do 17546 (KR)**

(74) Representative: **Casalonga**
**Casalonga & Partners**
**Bayerstraße 71/73**
**80335 München (DE)**

(54) **BIOLOGICAL MEASUREMENT DEVICE AND BIOLOGICAL DEVICE INCLUDING SAME**

(57)      Disclosed is a biological measurement device. The biological measurement device includes a first plate including a first transmission window; an electrode structure disposed on the first plate and spaced apart from the first window; a chamber plate disposed over the electrode structure and the first plate, the chamber plate including for exposing a portion of the electrode structure and the first transmission window; a second plate disposed over the chamber plate, the second plate including a second transmission window facing the first transmission window; a light emitting unit positioned either over the second transmission window and emitting light toward the second transmission window; and a light receiving unit disposed under the first transmission window and being configured to receive the light which the light emitting unit supplies to pass through the blood sample.

[FIG. 1]

## Description

### TECHNICAL FIELD

The present invention relates to a biological measurement device capable of measuring health information from a blood sample and a biological measuring apparatus including the same, which are mainly applied to a small-sized diagnostic equipment.

### BACKGROUND ART

On-site diagnostic equipment, which has been used in the vicinity of or near patients, enables faster measurement than existing research or automated equipment does, and thus the time leading to the diagnosis and subsequent prescription of the patient, thus, which may make TAT (turn-around time) reduced. Among the on-site diagnostic equipment, those who specialize in their functions more and reduce their size and cost are called small-sized diagnostic equipments, and a representative one of the equipments is called to be a personal blood glucose meter. The small-sized diagnostic equipments have the advantage of being easy to use and carry them. Further, the small-sized diagnostic equipments may be required to spend much lower measurement costs than other instruments. These advantages may be appropriate to the present medical system, as a Ubiquitous health care system develops and the medical equipments are increasingly miniaturized.

One area where such small-sized diagnostic equipments are used is for anemia diagnosis. Anemia disease may be classified into macrocytic anemia, normocytic normochromic anemia, and microcytic hypochromic anemia according to types of red blood cells. In order to classifying anemia, it may be necessary to measure a mean corpuscular volume (MCV) and a mean corpuscular hemoglobin concentration (MCHC) value. For measuring MCV and MCHC value, blood erythrocyte concentration, hematocrit, and blood hemoglobin concentration should be measured in advance.

However, the conventional small-sized diagnostic equipments may simply determine only whether the patient catches the anemia and cannot determine a type of anemia, and as a result, it should be required for the doctor to take an additional blood test against the patient. The biggest advantages of the small-sized diagnostic equipment is that it reduces the diagnosis and treatment time, TAT, of the disease, but existing small-sized diagnostic equipments for diagnosing anemia can only determine the presence of anemia still by measuring only a concentration of hemoglobin or hematocrit. Further, the existing small-sized diagnostic equipments for diagnosing anemia cannot measures both the concentration of hemoglobin and hematocrit at the same time. There is also no small on-site diagnostic equipment that simultaneously measures both anemia and blood glucose level of pregnant women and a patient of a second type of diabetic.

The present invention relates to a biological measurement device capable of complexly measuring hematocrit and the hemoglobin concentration value, and further measuring blood glucose levels in order to come over the shortcomings of the conventional small sized diagnostic equipments for measuring anemia.

### DETAILED DESCRIPTION OF THE INVENTION

### TECHNICAL PROBLEM

The present invention has one object to measure the level of hematocrit and hemoglobin value of possible pregnancy women and adolescent patients at the same time to help determine a type of anemia, and to measure degrees of anemia and blood glucose levels of pregnant women and type 2 diabetes patients simultaneously to provides the pregnant women and the type 2 diabetes patients with the rapid diagnosis and information on the treatment.

The present invention has another object to provide a biological measuring apparatus including the biological measurement device.

### TECHNICAL SOLUTION

According to some example embodiments, a biological measurement device includes a first plate including a first transmission window capable of transmitting light, the first plate having a first corner through which a blood sample is inserted; an electrode structure disposed on the first plate and spaced apart from the first window; a chamber plate disposed over the electrode structure and the first plate, the chamber plate including a first opening portion extending from a fifth corner corresponding to the first corner toward a six corner opposite to the fifth corner, to expose a portion of the electrode structure and the first transmission window; and a second plate disposed over the chamber plate, the second plate including a second transmission window facing the first transmission window.

In an example embodiment, the electrode structure may include a first electrode structure having a first operating electrode to which a first operating voltage is applied, and a first auxiliary electrode extending in parallel with the fist operating electrode.

In an example embodiment, the electrode structure may include a second electrode structure having a second operating electrode, a second auxiliary electrode disposed adjacent to the second operating electrode, and a reagent layer covering at least one of the second operating electrode and the second auxiliary electrode, and including an electron transfer medium and a glycosylating enzyme.

In an example embodiment, the electrode structure may include a first electrode structure having a first operating electrode interposed between the first corner of the first plate and the first transmission window, to which a first operating voltage is applied, and a first auxiliary electrode interposed between the first operating electrode and the first transmission window and in parallel with the fist operating electrode; and a second electrode structure having a second operating electrode disposed in parallel with the first operating electrode with the first transmission window interposed therebetween , a second auxiliary electrode disposed adjacent to the second operating electrode, and a reagent layer covering either the second operating electrode or the second auxiliary electrode, and including an electron transfer medium and a glycosylating enzyme.

In an example embodiment, the biological measurement device may further include a sealing layer interposed between the chamber plate and the electrode structure, the sealing layer including a second opening portion corresponding to first opening portion, and being disposed to at least partially cover the electrode structure such that the sealing layer prevents the blood sample from leaking between the chamber plate and the electrode structures. Here, the sealing layer may include an insulating polymer material, and has a thickness lower than that of the chamber plate.

In an example embodiment, the first operating electrode may include a first electrode portion extending in a direction perpendicular to a direction along which the blood sample is injected between the first corner of the first plate and the first transmission window, and a second electrode portion being bent from an end portion of the first electrode portion, the second operating electrode may include a fifth electrode portion extending in parallel with the first electrode portion of the first operating electrode such that the first transmission window is interposed between the first electrode portion and the fifth electrode portion, and a sixth electrode portion being bent from an end portion of the fifth electrode portion, and the first plate further includes a first blocking rib extending between the second electrode portion and the sixth electrode portion, and protruding from an upper surface of the first plate. Here, the first auxiliary electrode may further include a third electrode portion disposed between the first corner of the first plate and the first transmission window and in parallel with the first electrode portion, and a fourth electrode portion being bent from an end portion of the third electrode portion, the second auxiliary electrode may further include a seventh electrode portion being adjacent to the fifth electrode portion and extending in parallel with the fifth electrode portion, and an eighth electrode portion being bent and extending from an end portion of the seventh electrode portion and being spaced apart from the fourth electrode portion, and the first plate further includes a second blocking rib extending between the fourth electrode portion and the eighth electrode portion, and protruding from an upper surface of the first plate. Further, the first blocking rib may have a thicknesses equal to or smaller than those of the second and the sixth electrode portions, and the second blocking rib may have a thicknesses equal to or smaller than those of the fourth and the eighth electrode portions.

In an example embodiment, the light emitting unit may include a green light source generating light having a green light wavelength and a infrared light source generating light having a infrared light wavelength

In an example embodiment, the biological measurement device may further include an anti-reflection film disposed on at least one of a lower surface and an upper surface of the first plate and on at least one of a lower surface and an upper surface of the second plate.

According to some example embodiments, a biological measuring apparatus includes a biological measurement device generating first to third electric signals corresponding to information on a hematocrit, a hemoglobin concentration, and a blood glucose level of the blood sample, respectively, a control device including a processor unit receiving the first to third electric signals and generating information about hematocrit and hemoglobin concentration in the blood sample, and a raw information about blood glucose level in the blood sample; an anemia-type analyzing unit receiving information on the hematocrit and the hemoglobin concentration from the processor unit to generate information on a type of anemia based on the information which the processor unit provides; and a blood glucose level correcting unit receiving information on the hematocrit and raw information on the blood glucose level from the processor unit to correct the raw information on the blood glucose level using the information on the hematocrit to generate corrected information on the blood glucose level, and a display device being configured to display information on at least one of hematocrit, hemoglobin concentration of the blood sample, corrected information on blood glucose concentration, and the type of anemia.

In an example embodiment, the anemia-type analyzing unit may calculate an MCHC (Mean corpuscular hemoglobin concentration) value representing an average red blood cell hemoglobin concentration using Equation 1 from the information on the hematocrit and the hemoglobin concentration to generate information on a type of anemia.

Equation I

$$MCHC\,[g/dL] = \frac{Hb\,[g/dL]}{Hct\,[\%]}\,\text{s}100$$

Here, 'Hb' represents a hemoglobin concentration value in the blood sample, 'Hct' represents a hematocrit.

In an example embodiment, the biological measurement device may include a first plate including a first transmission window capable of transmitting light, the first plate having a first corner through which a blood sample is inserted; a first electrode structure disposed on the first plate, and interposed between the first corner of the first plate and the first transmission window, the first electrode structure including a first operating electrode to which a first operating voltage is applied, and a first auxiliary electrode being interposed between the operating electrode and the first transmission window and extending in parallel with the fist operating electrode; a second electrode structure having a second operating electrode disposed on the first plate and in parallel with the first operating electrode with the first transmission window interposed therebetween, a second auxiliary electrode disposed adjacent to the second operating electrode, and a reagent layer covering at least one of the second operating electrode and the second auxiliary electrode, and including an electron transfer medium and a glycosylating enzyme; a chamber plate disposed over the first and the second the electrode structures, the chamber plate including a first opening portion extending from a first corner corresponding to the first corner of the first plate toward a second corner opposite to the first corner, to expose the first and the second operating electrodes, the first and the second auxiliary electrodes and the first transmission window; a second plate disposed over the chamber plate, the second plate including a second transmission window facing the first transmission window; a light emitting unit positioned either over the second transmission window to emit light toward the second transmission window or under the first transmission window to emit light toward the first transmission window; and a light receiving unit interposed between the first transmission window and the second transmission window to face the light emitting unit, the light receiving unit being configured to receive the light which the light emitting unit supplies to pass through the blood sample.

In an example embodiment, the first electric signal may correspond to a current value generated between the first operating electrode and the first auxiliary electrode by the blood sample, the second electric signal corresponds to an amount of light passing through the blood sample and being incident onto the light receiving unit, and the third electric signal corresponds to a current which the blood sample generates and flows between the second operating electrode and the second auxiliary electrode.

In an example embodiment, the control device may control an operation of the light emitting unit such that the light emitting unit irradiates light to the blood sample when receiving the third electrical signal from the biological measurement device.

According to some example embodiments, a biological measuring apparatus includes a biological measurement device generating a first electric signal corresponding to information on a hematocrit, and a second electric signal corresponding to information on a hemoglobin concentration; a control device including a processor unit receiving the first and the second electric signals and generating raw information about hematocrit and hemoglobin concentration in the blood sample, and an anemia-type analyzing unit receiving the information on the hematocrit and the hemoglobin concentration from the processor unit to generate information on a type of anemia based on the information which the processor unit provides; and a display device being configured to display information on at least one of hematocrit, hemoglobin concentration of the blood sample, and the type of anemia.

According to some example embodiments, a biological measuring apparatus includes a biological measurement device generating a first electric signal corresponding to information on a hematocrit in the blood sample, and a second electric signal corresponding to information on a blood glucose level in the blood sample; a control device including a processor unit receiving the first and the second electric signals and generating information about hematocrit and a raw information about blood glucose level in the blood sample, and a blood glucose level correcting unit receiving information on the hematocrit and raw information on the blood glucose level from the processor unit to correct the raw information on the blood glucose level using the information on the hematocrit to generate corrected information on the blood glucose level; and a display device being configured to display information on at least one of hematocrit and corrected information on blood glucose concentration.

**ADVANTAGEOUS EFFECTS**

According to the biological measuring apparatus of the present invention, since the control unit includes a blood glucose concentration measurement value correcting unit for correcting the blood glucose concentration information measured by the blood glucose concentration measuring unit using information on hematocrit measured by the hematocrit measuring unit. Thus, accurate blood glucose concentrations may be determined from a blood sample. In addition, the control unit includes an anemia type analyzing unit for analyzing the type of anemia using the information on hematocrit measured by the hematocrit measuring unit and the information on the hemoglobin concentration value measured by the hemoglobin concentration measuring unit to provide more specific and accurate health information for to a user.

## BRIEF DESCRIPTION OF THE DRAWINGS

FIG. 1 is an exploded perspective view illustrating a biological measurement device in accordance with an example embodiment of the present invention.

FIG. 2 is a partial cross-sectional view illustrating the biological measurement device in FIG. 1.

FIGS. 3 and 4 are a plan view and a cross-sectional view illustrating another example of the first plate shown in FIG. 1, respectively.

FIG. 5 is a block diagram illustrating a biological measuring apparatus in accordance with an embodiment of the present invention.

## BEST MODE OF THE INVENTION

Hereinafter, specific embodiments will be described in detail with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Like reference numerals refer to like elements throughout. In the figures, the dimensions of layers and regions are exaggerated for clarity of illustration.

The terminology used herein is for the purpose of describing particular example embodiments only and is not intended to be limiting of the present inventive concept. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

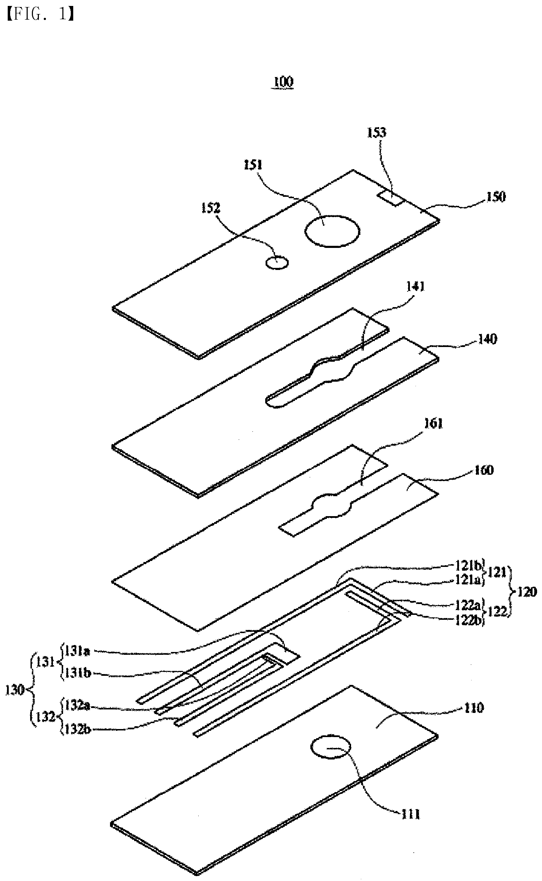

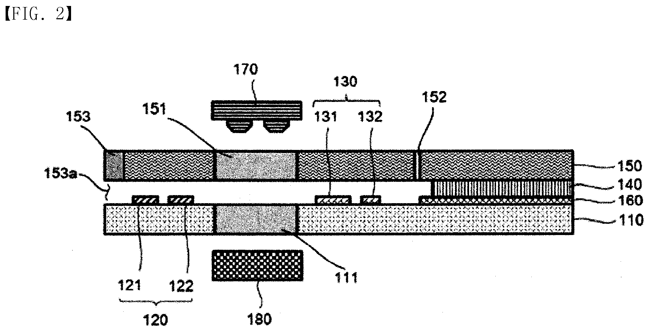

FIG. 1 is an exploded perspective view illustrating a biological measurement device in accordance with an example embodiment of the present invention. FIG. 2 is a partial cross-sectional view illustrating the biological measurement device shown in FIG. 1.

Referring to FIGS. 1 and 2, a biological measurement device 100 in accordance with an example embodiment of the present invention includes a first plate 110, a first electrode structure 120, a second electrode structure 130, a chamber plate 140 and a second plate 150.

The first plate 110 may have a plate form including a flat upper face and a flat lower face. The first plate 110 may include a polymer material such as polyethylene terephthalate (PET), polyethylene (PE), etc. A shape of the first plate 110 is not particularly limited, but for describe the first plate 110 conveniently, the first plate 110 having a rectangular shape will be described below as one of examples. That is, the first plate 110 includes a first corner, a second corner facing the first corner, and a third and a fourth corners connecting end portions of the first and second corners with each other. An injection hole 153a is formed on the first corner such that a blood sample is injected through the injection hole 153a. Thus, the first plate 110 may have a rectangular plate shape.

The first plate 110 may include a first transmission window 111 through which light may be transmitted. The first transmission window 111 may be formed at a predetermined position to be spaced apart from the first corner toward the second corner by a predetermined interval. In an example embodiment, an anti-reflection film (not shown) may be formed on a lower surface of the first plate 110. Therefore, either light irradiated by a light emitting unit 170 or external light may be prevented from reflecting from the lower surface of the first plate 110 except for the first transmission window 111 to minimize interference with incident light. A material or structure of the antireflection film is not particularly limited, and known antireflection materials and structures may be applied without limitation. Meanwhile, the anti-reflection film may be further formed on an upper surface of the first plate 110 in order to minimize interference of light due to reflection of light which may occur in the biological measurement device 100.

The first electrode structure 120 and the second electrode structure 130 may be disposed on the upper surface of the first plate 110. A material of each the first and second electrode structures 120 and 130 is not particularly limited as long as it is an electrically conductive material, and a method of forming the first and the second electrode structures 120 and 130 is not particularly limited. In some example embodiments, the first and second electrode structures 120 and 130 may be either formed by adhering a conductive thin film to the upper surface of the first plate 110, or formed by depositing or printing a conductive material.

The first electrode structure 120 may include a first operating electrode 121 and a first auxiliary electrode 122. The first electrode structure 120 may measure hematocrit through an electrical method, which is a volume fraction of red blood cells which occupies a blood sample supplied

In an example embodiment, the first operating electrode 121 includes a first electrode portion 121a and a second electrode portion 121b. The first electrode portion 121a extends in a direction perpendicular to a direction in which the blood sample is injected, and is disposed between the first corner of the first plate 110 and the first transmission window 111. The second electrode portion 121b may be bent from an end portion of the first electrode portion 121a and may extend from the end portion of the first electrode portion 121a to the second corner along the third corner of the first plate 110.

The first auxiliary electrode 122 includes a third electrode portion 122a and a fourth electrode portion 122b. The third electrode portion 122a is spaced apart from the first electrode portion 121a and extends in parallel with the first electrode portion 121a. The fourth electrode portion 122b is bent from an end portion of the third electrode portion 122a. The fourth electrode portion 122b extends from the end portion of the third electrode portion 122a to the second corner of the first plate 110 along the fourth corner of the first plate 110.

In order to measure the hematocrit value, a first operating voltage may be applied to the first operating electrode 121, whereas the first auxiliary electrode 122 may be grounded. When the blood sample contacts the first electrode portion 121a of the first operating electrode 121 and the third electrode portion 122a of the first auxiliary electrode 122, a current flows between the first operating electrode 121 and the first auxiliary electrode 122, and the hematocrit value of the blood sample can be measured by measuring the current. In general, since a red blood cell has a highest impedance value among those of blood components, the higher a volume ratio of the red blood cells in the blood sample, the higher the impedance value of the blood sample. Therefore, when either a current value or the voltage value between the first operating electrode 121 and the first auxiliary electrode 122 caused by the blood sample is measured, the hematocrit value of the blood sample may be easily calculated.

The second electrode structure 130 may include a second operating electrode 131 and a second auxiliary electrode 132, and may measure a blood glucose level in the blood sample by an electrochemical method.

In an example embodiment, the second operating electrode 131 includes a fifth electrode portion 131a and a sixth electrode portion 131b. The fifth electrode portion 121a extends in parallel with the first electrode portion 121a of the first operating electrode 121 such that the first transmission window 111 is interposed between the first electrode portion 121a and the fifth electrode portion 131a. The sixth electrode portion 131b may be bent from an end portion of the fifth electrode portion 131a and may extend from the end portion of the fifth electrode portion 131a. The sixth electrode portion 131b may be spaced apart from the second electrode portion 121b of the first operating electrode 121 by a predetermined interval.

The second auxiliary electrode 132 includes a seventh electrode portion 132a and an eighth electrode portion 132b. The seventh electrode portion 132a is adjacent to the fifth electrode portion 131a of the second operating electrode 131 and extends in parallel with the fifth electrode portion 131a of the second operating electrode 131. The eighth electrode portion 132b is bent and extends from an end portion of the seventh electrode portion 132a. The eighth electrode portion 132b is spaced apart from the fourth electrode portion 122b of the first auxiliary electrode 122 by a predetermined interval.

In addition, either the fifth electrode portion 131a of the second operating electrode 131 or the seventh electrode portion 132a of the second auxiliary electrode 132 may include a reagent layer (not shown) having an electron transfer medium and a glycosylating enzyme. The glycosylating enzyme may reduce the electron transfer medium while oxidizing glucose in the blood sample into gluconic acid.

As the electron transfer medium and the glycosylation enzyme, known materials may be used without limitation. For example, the electron transfer medium includes hexaammineruthenium(III)chloride, potassium ferricyanide), potassium ferrocyanide, dimethylferrocene(DMF), ferricinium, ferocene monocarboxylic acid(FCOOH), 7,7,8,8-tetracyanoquino-dimethane(TCNQ), tetrathia fulvalene(TTF), nickelocene(Nc), N-methyl acidinium(NMA+), tetrathiatetracene(TTT), N-methylphenazinium (NMP+), hydroquinone, 3-dimethylaminobenzoic acid(MBTHDMAB), 3-methyl-2-benzothiozolinone hydrazone, 2-methoxy-4-allylphenol, 4-aminoantipyrin(AAP), dimethylaniline, 4-aminoantipyrene, 4-methoxynaphthol, 3,3',5,5'-tetramethyl benzidine(TMB), 2,2-azino- di-[3-ethyl-benzthiazoline sulfonate]), odianisidine, o-toluidine, 2,4-dichlorophenol, 4-amino phenazone, benzidine, prussian blue, Bipyridine-osmium Complex Compound, etc. The glycosylation enzyme includes flavin adenine dinucleotide-glucose dehydrogenase (FAD-GDH), nicotinamide adenine dinucleotide-glucose dehydrogenase (NAD-GDH), Pyrroloquinoline quinone-glucose dehydrogenase (PQQ-GDH), glucose oxidase (GOx), etc.

According to the reagent layer as described above, when the blood sample contacts the second operating electrode 131, the oxidation potential is generated in the second operating electrode 131 due to the electron transfer medium. As a result, when the blood sample is in contact with the second operating electrode 131 and the second auxiliary electrode 132 at the same time, an electric current, generated by an electric potential which may occur in the oxidation of the blood sample, may flow between the second operating electrode 131 and the second auxiliary electrode 132. Therefore, the blood glucose level contained in the blood sample may be easily measured by measuring a value of the electric current which flows between the second operating electrode 131 and the second auxiliary electrode 132, which may be caused by the redox reaction as described above.

The chamber plate 140 is disposed over the first plate 110 on which the first and second electrode structures 120 and 130 are disposed. The chamber plate 140 may be formed using a polymer such as polyethylene terephthalate (PET) and polyethylene (PE).

The chamber plate 140 may define a space in which the blood sample is injected together with both the first plate 110 and the second plate 150. In an example embodiment, the chamber plate 140 has a fifth corner, a sixth corner, a seven corner and an eighth corner. The fifth corner has a width equal to the first corner of the first plate 110 and overlaps the first corner of the first plate 110. The sixth corner faces the fifth corner to be spaced apart from the fifth corner. The seventh and eighth corners connect both end portions of the fifth corner and the sixth corner with each other. The chamber plate 140 may have a rectangular shape. The chamber plate 140 may include a first opening portion 141 which extends from the fifth corner into which the blood sample is injected toward the sixth corner. The first opening portion 141 may expose a first electrode portion 121a of the first operating electrode 121, a third electrode portion 122a of the first auxiliary electrode 122, and the first transmission window 111 of the first plate 110., the fifth electrode portion 131a of the second operating electrode 131, and the seventh electrode portion 132a of the second auxiliary electrode 132.

Although a thickness of the chamber plate 140 and a width of the first opening 141 are not particularly limited, the thickness of the chamber plate 140 and the width of the first opening 141 may be adjusted such that the blood sample may be injected into the first opening 141 through capillary phenomena. However, in order for the blood sample to be injected into the first opening 141, the thickness of the chamber plate 140 may be greater than the thickness of the first and second electrode structures 120 and 130.

The second plate 150 may be disposed on the chamber plate 140, and as described above, the second plate 150 may define the space in which the blood sample is injected together with the first plate 110 and the chamber plate 140.

The second plate 150 may be formed of a polymer material such as polyethylene terephthalate (PET) or polyethylene (PE), and may have a plate shape having a flat upper surface and a lower surface. In one embodiment, the second plate 150 has a ninth corner, a tenth corner, an eleventh corner and a twelfth corner. The ninth corner has a width equal to the first corner of the first plate 110 and overlaps the first corner of the first plate 110. The tenth corner faces the ninth corner to be spaced apart from the ninth corner. The eleventh and twelfth corners connect both end portions of the ninth corner and the tenth corner with each other. Thus, the second plate 150 may have a rectangular shape.

In an example embodiment, the second plate 150 may include a second transmission window 151 and an outlet 152. The second transmission window 151 faces the first transmission window 111 of the first plate 110 to transmit light. The outlet 152 may be configured to discharge air from the space into which the blood sample is injected. In addition, the second plate 150 may include a transparent window 153 exposing the space into which the blood sample is injected. The transparent window 153 may be spaced apart from the ninth corner by a predetermined interval along the first opening 141 of the chamber plate 140.

In an example embodiment, an anti-reflection film (not shown) is formed on an upper surface of the second plate 150. The anti-reflection film may prevent the light irradiated by the light emitting unit 170 or external light from reflecting from the upper surface of the second plate 150 except for the second transmission window 151 to minimize interference with incident light. The material or structure of the anti-reflection film is not particularly limited, and known antireflection materials and structures may be applied without limitation. Meanwhile, the anti-reflection film may be further formed on a lower surface of the second plate 150 in order to minimize interference with incident light, which may occur due to reflection of light inside of the biological measurement device 100.

The biological measurement device 100 according to an example embodiment of the present invention may further include a sealing layer 160. The sealing layer 160 may be interposed between the chamber plate 140 and the first and second electrode structures 120 and 130. The sealing layer 160 is disposed to at least partially cover the first and the second operating electrodes 121 and 131 and the first and second auxiliary electrodes 122 and 132. The sealing layer 160 may be formed of an insulating polymer material, and may have a thickness lower than that of the chamber plate 140.

The sealing layer 160 may prevent either the first operating electrode 121 and the second operating electrode 131 or the first auxiliary electrode 122 and the second auxiliary electrode 132 from being electrically connected to each other, which may occur due to the blood sample while the blood sample leaks through a space between the chamber plate 140 and the first and second electrode structures 120 and 130.

In some example embodiments, the sealing layer 160 may include a second opening portion 161 corresponding to the first opening portion 141 of the chamber plate 140. The second opening portion 161 may expose the first electrode portion 121a of the first operating electrode 121, the third electrode portion 122a of the first auxiliary electrode 122, the first transmission window 111 of the first plate 110. T, the fifth electrode portion 131a of the second operating electrode 131, and the seventh electrode portion 132a of the second auxiliary electrode 132.

The biological measurement device 100 according to an example embodiment of the present invention may further include a light emitting unit 170 and a light receiving unit 180. The light emitting unit 170 may be positioned over the second transmission window 151 of the second plate 150 to emit light to the blood sample through the second transmission window 151. Alternatively, the light emitting unit 170 may be positioned under the first transmission window 111 of the first plate 110 to emit light to the blood sample through the first transmission window 111. The light receiving unit 180 may be interposed between the first transmission window 111 of the first plate 110 and the second transmission window 151 of the second plate 150 to face the light emitting unit 170. The light receiving unit 180 may receive the light which is supplied thereinto after passing through the blood sample and may generate an electrical signal corresponding to an amount of the received light.

The biological measurement device 100 according to some example embodiments of the present invention may measure a concentration of hemoglobin contained in the blood sample using the light emitting unit 170 and the light receiving unit 180. In general, hemoglobin absorbs light having a specific wavelength. Thus, an amount of light at which arrived to the light receiving unit 180 may vary according to the concentration of hemoglobin contained in the blood sample, when the light emitting unit 170 emits the light having the wavelength capable of being absorbed by hemoglobin. Therefore, as the amount of the received light may change, the biological measurement device 100 may measure the concentration of hemoglobin in the blood sample by using a value of the electric signal which may vary according to the amount of the light received by the light receiving unit 180.

In an example embodiment, the light emitting unit 170 may include a plurality of light sources for generating light having various wavelengths different from each other. For example, the light emitting unit 170 may include a green light source for generating light of a green wavelength and an infrared light source for generating light of an infrared wavelength. The green light source may be used to analyze the concentration of hemoglobin based on an absorption spectrometry, and the infrared light source may be used for correcting the influence by high concentrations of white blood cells and lipids in the blood sample.

Meanwhile, the light emitting unit 170 may irradiate light to the blood sample when the blood sample reaches to the second operating electrode 131 and the second auxiliary electrode 132 such that a current flow from the second operating electrode 131 to the second auxiliary electrode 132 may be detected.

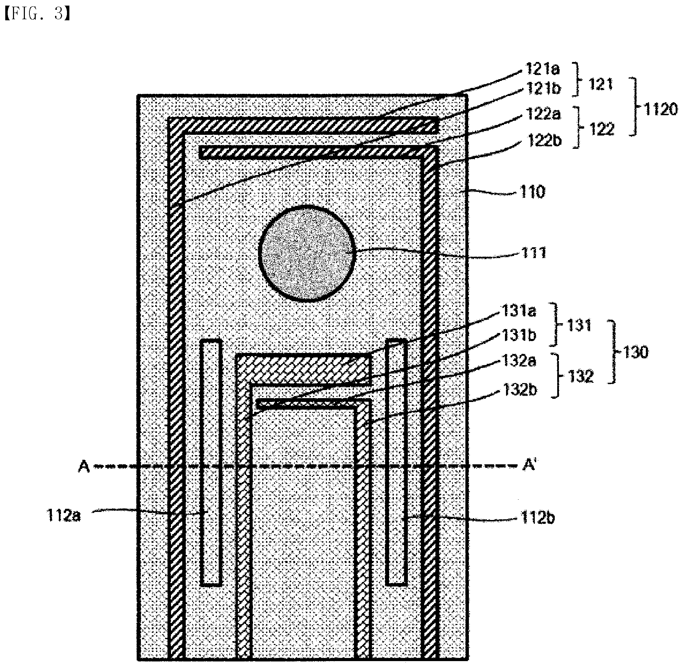

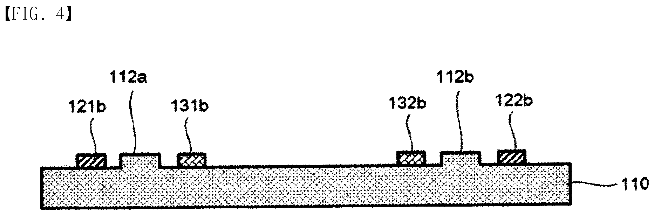

FIGS. 3 and 4 are a plan view and a cross-sectional view illustrating another example of the first plate shown in FIG. 1, respectively.

Referring to FIGS. 3 and 4 together with FIGS. 1 and 2, according to an example embodiment of the present invention, the first plate 110 may further include a first blocking barrier rib 112a and a second blocking barrier rib 112b. The first blocking rib 112a extends between the second electrode portion 121b of the first operating electrode 121 and the sixth electrode portion 131b of the second operating electrode 131, and protrudes from an upper surface of the first plate 110. The second blocking rib 112a extends between the fourth electrode portion 122b of the first auxiliary electrode 122 and the eighth electrode portion 132b of the second auxiliary electrode 132, and protrudes from the upper surface of the first plate 110. Therefore, the first and the second blocking ribs 112a and 112b may prevent either the first operating electrode 121 and the second operating electrode 131 or the first auxiliary electrode 122 and the second auxiliary electrode 132 from being electrically connected with each other, which may occur due to the leaked blood sample.

In order to prevent a space in which the blood sample is leaked from being defined between the first and second blocking ribs 112a and 112b, the first and second blocking ribs 112a and 112b may have the thicknesses equal to or smaller than the those of the first and the second electrode structures 120 and 130.

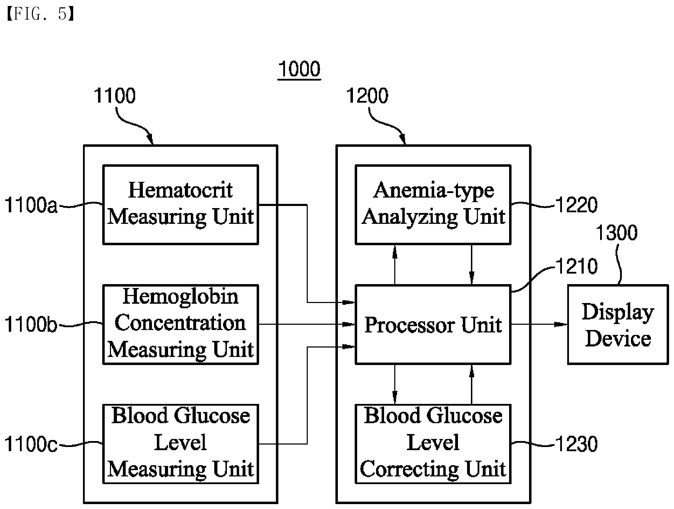

FIG. 5 is a block diagram illustrating a biological measuring apparatus in accordance with an embodiment of the present invention.

Referring to FIG. 5 together with FIGS. 1 to 4, a biological measuring apparatus 1000 in accordance with an embodiment of the present invention include a biological measurement device 1100, a control device 1200 and a display device 1300.

The biological measurement device 1100 may include a hematocrit measuring unit 1100a, a hemoglobin concentration measuring unit 1100b, and a blood glucose level measuring unit 1100c. In an example embodiment, the biological measurement device 1100 is substantially the same as the biological measurement device 100 described with reference to FIGS. 1 to 4, and thus descriptions thereof will be omitted.

The hematocrit measuring unit 1100a may measure hematocrit which is a volume fraction of red blood cells in a blood sample by an electrical method.

In an example embodiment, the hematocrit measuring unit 1100a may include a first operating electrode 121 and a first auxiliary electrode 122 of the first electrode structure 120. A first operating voltage may be applied to the first operating electrode 121. When the blood sample simultaneously contacts the first operating electrode 121 and the first auxiliary electrode 122, the hematocrit measuring unit 1100a may generate a first electric signal corresponding to a current value or a voltage value generated between the first operating electrode 121 and the first auxiliary electrode 122.

As described above, since the red blood cells of the blood components have the highest impedance value, the higher the red blood cell volume ratio included in the blood samples, the higher the impedance value of the blood sample. Thus, the first electric signal corresponding to the current value or the voltage value measured between the first operating electrode 121 and the first auxiliary electrode 122 due to the blood sample may be related to hematocrit information about the blood sample.

The hemoglobin concentration measuring unit 1100b may measure a concentration of hemoglobin contained in the blood sample by an optical method.

In an example embodiment, the hemoglobin concentration measuring unit 1100b includes a light emitting unit 170 capable of emitting light toward the blood sample, and a light receiving unit 180 capable of receiving light which the light emitting unit 170 irradiates toward the blood sample and then passes through the blood sample. The hemoglobin concentration measuring unit 1100b may generate a second electric signal corresponding to an amount of light passing through the blood sample and being incident onto the light receiving unit 180.

As described above, since hemoglobin absorbs light having a specific wavelength, an amount of light at which arrived to the light receiving unit 180 may vary according to the concentration of hemoglobin contained in the blood sample, when the light emitting unit 170 emits the light having the wavelength capable of being absorbed by hemoglobin. Therefore, the hemoglobin concentration measuring unit 110b may generate the second electric signal having information on the value of hemoglobin concentration included in the blood sample.

On the other hand, the control device 1200 may control the light emitting unit 170 for irradiating light toward the blood sample. When the blood sample reaches to the second operating electrode 131 and the second auxiliary electrode 132, the blood glucose level measuring unit 1100c detects a current which flows from the second operating electrode 131 to the second auxiliary electrode 132.

The blood glucose level measuring unit 1100c may measure a level of the blood glucose contained in the blood sample through an electrochemical method.

In some example embodiments, the blood glucose level measuring unit 1100c includes a second operation electrode 131 and a second auxiliary electrode 132 included in the second electrode structure 130. Either the second operation electrode 131 or the second auxiliary electrode 132 may include a reagent layer (not shown) including an electron transfer medium and a glycosylating enzyme. When the blood sample simultaneously contacts the second operating electrode 131 and the second auxiliary electrode 132, the blood glucose level measuring unit 1100c may generate a third electric signal corresponding to a current which flows between the second operating electrode 131 and the second auxiliary electrode 132. The glycosylating enzyme may reduce the electron transfer medium while oxidizing glucose in the blood sample into gluconic acid. Therefore, when the blood sample contacts the second operating electrode 131, the oxidation potential is generated in the second operating electrode 131 due to the electron transfer medium. As a result, an electric current, generated by an electric potential which may occur in the oxidation of the blood sample, may flow between the second operating electrode 131 and the second auxiliary electrode 132. Thus, the blood glucose level contained in the blood sample may be easily measured by measuring a value of the electric current which flows between the second operating electrode 131 and the second auxiliary electrode 132, which may be caused by the redox reaction as described above.

The control device 1200 is electrically connected to the biological measurement device 1100 and generates information about the hematocrit, the hemoglobin concentration, and the blood glucose level of the blood sample, a type of anemia, etc. based on the first to third electric signals provided by the biological measurement device 1100.

In an example embodiment, the control device 1200 may include a processor unit 1210, an anemia-type analyzing unit 1220, and a blood glucose level correcting unit 1230.

The processor unit 1210 is electrically connected to the hematocrit measuring unit 1100a, the hemoglobin concentration measuring unit 1100b, and the blood glucose level measuring unit 1100c, and is provided with the first to third electrical signals provided therefrom. The processor unit 1210 may compare the first to third electrical signals with pre-stored information on, for example, change in hematocrit with respect to the impedance change, hemoglobin concentration information in accordance with the change in the amount of light incident on the light-receiving unit, a blood glucose level according to the current change, respectively, to generate raw information about hematocrit, hemoglobin concentrations and blood glucose level of the blood sample.

The anemia-type analyzing unit 1220 may receive information on the hematocrit and the hemoglobin concentration from the processor unit 1210, and generate information on the type of anemia based on the information which the processor unit 120 provides. In an example embodiment, the anemia-type analyzomg unit 1220 generates an MCHC (Mean corpuscular hemoglobin concentration) value representing an average red blood cell hemoglobin concentration using Equation 1 below, and determine the type of the anemia based on the generated MCHC value information

Equation I

$$MCHC[g/dL] = \frac{Hb[g/dL]}{Hct[\%]} \leq 100$$

In Equation 1, 'Hb' represents a homoglobin concentration value in the blood sample, 'Hct' represents a hematocrit.

In an example embodiment, the anemia type analysis unit 1220 diagnoses hypochromia when the MCHC value calculated from the information on the hematocrit and the information on the hemoglobin concentration provided from the processor unit 1210 is equal or less than about 30, and generates information corresponding to the anemia (hypochromia). When the MCHC value is in a range of about 31 to about 35, the anemia type analysis unit 1220 classifies the anemia type as normochromia and generates information corresponding to the anemia (hypochromia).

The blood glucose level correcting unit 1230 is provided with information on the hematocrit and raw information on the blood glucose level from the processor unit 1210, and corrects the raw information on the blood glucose level using the information on the hematocrit to generate corrected information on the blood glucose level. In general, since hematocrit affects the impedance of the blood sample, blood samples having the same blood glucose level to each other also show different current values in accordance with the hematocrit in the blood sample. Therefore, in order to determine the exact blood glucose level, it is necessary to correct the raw information on the blood glucose level using the information on the hematocrit of the blood sample. According to example embodiments of the present invention, the blood glucose level correcting unit 1230 may perform such a correction process to generate accurate blood glucose levels.

The display unit 1300 is electrically connected to the control unit 1200, for example, the processor unit 1210, and may display information on at least one of hematocrit and hemoglobin concentration of the blood sample, corrected information on blood glucose concentration, and the type of anemia, provided by the control device 1200. As the display unit 1300, a known display device may be applied without limitation.

According to the biological measurement device and the biological-measuring apparatus including the biological measurement device of the present invention, since the information on the hematocrit and the blood glucose level are simultaneously measured, the information on the blood glucose level may be corrected by using the hematocrit information, thereby correcting the information on the blood glucose level from the blood sample to accurately measure the blood glucose level from the blood sample.

In addition, since the information on hematocrit and hemoglobin concentration may be measured at the same time, the information can be combined to analyze the type of anemia, thereby providing more specific and accurate health information to a user.

The foregoing is illustrative of the present teachings and is not to be construed as limiting thereof. Although a few exemplary embodiments have been described, those skilled in the art will readily appreciate from the foregoing that many modifications are possible in the exemplary embodiments without materially departing from the novel teachings and advantages of the present disclosure of invention. Accordingly, all such modifications are intended to be included within the scope of the present teachings. In the claims, means-plus-function clauses are intended to cover the structures described herein as performing the recited function and not only structural equivalents but also functionally equivalent structures.

**Claims**

1. A biological measurement device, comprising:

   a first plate including a first transmission window capable of transmitting light, the first plate having a first corner through which a blood sample is inserted;
   an electrode structure disposed on the first plate and spaced apart from the first window;
   a chamber plate disposed over the electrode structure and the first plate, the chamber plate including a first opening portion extending from a fifth corner corresponding to the first corner toward a six corner opposite to the fifth corner, to expose a portion of the electrode structure and the first transmission window; and
   a second plate disposed over the chamber plate, the second plate including a second transmission window facing the first transmission window.

2. The biological measurement device of claim 1, wherein the electrode structure includes a first electrode structure having a first operating electrode to which a first operating voltage is applied, and a first auxiliary electrode extending in parallel with the fist operating electrode.

3. The biological measurement device of claim 1, wherein the electrode structure includes a second electrode structure having a second operating electrode, a second auxiliary electrode disposed adjacent to the second operating electrode, and a reagent layer covering at least one of the second operating electrode and the second auxiliary electrode, and including an electron transfer medium and a glycosylating enzyme.

4. The biological measurement device of claim 1, wherein the electrode structure comprises:

a first electrode structure having a first operating electrode interposed between the first corner of the first plate and the first transmission window, to which a first operating voltage is applied, and a first auxiliary electrode interposed between the first operating electrode and the first transmission window and in parallel with the fist operating electrode; and
a second electrode structure having a second operating electrode disposed in parallel with the first operating electrode with the first transmission window interposed therebetween , a second auxiliary electrode disposed adjacent to the second operating electrode, and a reagent layer covering either the second operating electrode or the second auxiliary electrode, and including an electron transfer medium and a glycosylating enzyme.

5. The biological measurement device of claim 1, further comprising:
a sealing layer interposed between the chamber plate and the electrode structure, the sealing layer including a second opening portion corresponding to first opening portion, and being disposed to at least partially cover the electrode structure such that the sealing layer prevents the blood sample from leaking between the chamber plate and the electrode structures.

6. The biological measurement device of claim 5, wherein the sealing layer includes an insulating polymer material, and has a thickness lower than that of the chamber plate.

7. The biological measurement device of claim 4, wherein
the first operating electrode includes a first electrode portion extending in a direction perpendicular to a direction along which the blood sample is injected between the first corner of the first plate and the first transmission window, and a second electrode portion being bent from an end portion of the first electrode portion,
the second operating electrode includes a fifth electrode portion extending in parallel with the first electrode portion of the first operating electrode such that the first transmission window is interposed between the first electrode portion and the fifth electrode portion, and a sixth electrode portion being bent from an end portion of the fifth electrode portion, and
the first plate further includes a first blocking rib extending between the second electrode portion and the sixth electrode portion, and protruding from an upper surface of the first plate.

8. The biological measurement device of claim 7, wherein
the first auxiliary electrode further includes a third electrode portion disposed between the first corner of the first plate and the first transmission window and in parallel with the first electrode portion, and a fourth electrode portion being bent from an end portion of the third electrode portion,
the second auxiliary electrode further includes a seventh electrode portion being adjacent to the fifth electrode portion and extending in parallel with the fifth electrode portion, and an eighth electrode portion being bent and extending from an end portion of the seventh electrode portion and being spaced apart from the fourth electrode portion, and
the first plate further includes a second blocking rib extending between the fourth electrode portion and the eighth electrode portion, and protruding from an upper surface of the first plate.

9. The biological measurement device of claim 8, wherein
the first blocking rib has a thicknesses equal to or smaller than those of the second and the sixth electrode portions, and
the second blocking rib has a thicknesses equal to or smaller than those of the fourth and the eighth electrode portions.

10. The biological measurement device of claim 1, further comprising an anti-reflection film disposed on at least one of a lower surface and an upper surface of the first plate and on at least one of a lower surface and an upper surface of the second plate.

11. A biological measuring apparatus comprising:

a biological measurement device generating first to third electric signals corresponding to informations on a hematocrit, a hemoglobin concentration, and a blood glucose level of the blood sample, respectively;
a control device including a processor unit receiving the first to third electric signals and generating informations about hematocrit and hemoglobin concentration in the blood sample, and a raw information about blood glucose level in the blood sample; an anemia-type analyzing unit receiving informations on the hematocrit and the hemoglobin concentration from the processor unit to generate information on a type of anemia based on the informations which the processor unit provides; and a blood glucose level correcting unit receiving information on the hematocrit and raw information on the blood glucose level from the processor unit to correct the raw information on the blood glucose level using the information on the hematocrit to generate corrected information on the blood glucose level; and
a display device being configured to display information on at least one of hematocrit, hemoglobin concentration of the blood sample, corrected information on blood glucose concentration, and the type of anemia.

12. The biological measuring apparatus of claim 11, wherein the anemia-type analyzing unit calculates an MCHC (Mean corpuscular hemoglobin concentration) value representing an average red blood cell hemoglobin concentration using Equation 1 from the informations on the hematocrit and the hemoglobin concentration to generate information on a type of anemia.

Equation I

$$MCHC[g/dL] = \frac{Hb[g/dL]}{Hct[\%]} \mathsf{S} 100$$

Here, 'Hb' represents a homoglobin concentration value in the blood sample, 'Hct' represents a hematocrit.

13. The biological measuring apparatus of claim 11, wherein the bilogoical measurement device comprises:

a first plate including a first transmission window capable of transmitting light, the first plate having a first corner through which a blood sample is inserted;
a first electrode structure disposed on the first plate, and interposed between the first corner of the first plate and the first transmission window, the first electrode structure including a first operating electrode to which a first operating voltage is applied, and a first auxiliary electrode being interposed between the operating electrode and the first transmission window and extending in parallel with the fist operating electrode;
a second electrode structure having a second operating electrode disposed on the first plate and in parallel with the first operating electrode with the first transmission window interposed therebetween, a second auxiliary electrode disposed adjacent to the second operating electrode, and a reagent layer covering at least one of the second operating electrode and the second auxiliary electrode, and including an electron transfer medium and a glycosylating enzyme;
a chamber plate disposed over the first and the second the electrode structures, the chamber plate including a first opening portion extending from a first corner corresponding to the first corner of the first plate toward a second corner opposite to the first corner, to expose the first and the second operating electrodes, the first and the second auxiliary electrodes and the first transmission window;
a second plate disposed over the chamber plate, the second plate including a second transmission window facing the first transmission window;
a light emitting unit positioned either over the second transmission window to emit light toward the second transmission window or under the first transmission window to emit light toward the first transmission window; and
a light receiving unit interposed between the first transmission window and the second transmission window to face the light emitting unit, the light receiving unit being configured to receive the light which the light emitting unit supplies to pass through the blood sample.

14. The biological measuring apparatus of claim 13, wherein
the first electric signal corresponds to a current value generated between the first operating electrode and the first auxiliary electrode by the blood sample,
the second electric signal corresponds to an amount of light passing through the blood sample and being incident onto the light receiving unit, and
the third electric signal corresponds to a current which the blood sample generates and flows between the second operating electrode and the second auxiliary electrode.

15. The biological measuring apparatus of claim 14, wherein the control device controls an operation of the light emitting unit such that the light emitting unit irradiates light to the blood sample when receiving the third electrical signal from the biological measurement device.

16. A biological measuring apparatus comprising:

a biological measurement device generating a first electric signal corresponding to information on a hematocrit, and a second electric signal corresponding to information on a hemoglobin concentration;
a control device including a processor unit receiving the first and the second electric signals and generating raw informations about hematocrit and hemoglobin concentration in the blood sample, and an anemia-type analyzing unit receiving the informations on the hematocrit and the hemoglobin concentration from the processor unit to generate information on a type of anemia based on the informations which the processor unit provides; and
a display device being configured to display information on at least one of hematocrit, hemoglobin concentration of the blood sample, and the type of anemia.

17. A biological measuring apparatus comprising:

a biological measurement device generating a first electric signal corresponding to information on a hematocrit in the blood sample, and a second electric signal corresponding to information on a blood glucose level in the blood sample;
a control device including a processor unit receiving the first and the second electric signals and generating information about hematocrit and a raw information about blood glucose level in the blood sample, and a blood glucose level correcting unit receiving information on the hematocrit and raw information on the blood glucose level from the processor unit to correct the raw information on the blood glucose level using the information on the hematocrit to generate corrected information on the blood glucose level; and
a display device being configured to display information on at least one of hematocrit and corrected information on blood glucose concentration.

【FIG. 1】

【FIG. 2】

【FIG. 3】

【FIG. 4】

【FIG. 5】

## INTERNATIONAL SEARCH REPORT

International application No.

**PCT/KR2017/005252**

| A. CLASSIFICATION OF SUBJECT MATTER |
|---|
| *A61B 5/1468(2006.01)i, G01N 33/49(2006.01)i, G01N 33/487(2006.01)i, A61B 5/145(2006.01)i, A61B 5/00(2006.01)i* |
| According to International Patent Classification (IPC) or to both national classification and IPC |

| B. FIELDS SEARCHED |
|---|
| Minimum documentation searched (classification system followed by classification symbols) |
| A61B 5/1468; G01N 15/05; C12Q 1/00; G01N 33/49; G01N 33/48; G01N 27/327; G01N 33/72; G01N 27/416; G01N 33/487; A61B 5/145; A61B 5/00 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: light, transmission window, plate, electrode, hemoglobin, hematocrit, blood sugar, anemia, correction |

| C. DOCUMENTS CONSIDERED TO BE RELEVANT | | |
|---|---|---|
| Category* | Citation of document, with indication, where appropriate, of the relevant passages | Relevant to claim No. |
| X | US 2016-0320331 A1 (DELBIO, INC.) 03 November 2016<br>See paragraphs [13], [45] and figures 1A-1C. | 17 |
| Y | | 11,12,16 |
| A | | 1-10,13-15 |
| Y | WO 2007-128684 A1 (SIRE ANALYTICAL SYSTEMS SRL.) 15 November 2007<br>See page 2, lines 6-16. | 11,12,16 |
| A | US 2013-0062221 A1 (CAI et al.) 14 March 2013<br>See paragraphs [86]-[95] and figure 4. | 1-17 |
| A | US 2006-0051738 A1 (ZWEIG) 09 March 2006<br>See paragraph [122] and figure 4. | 1-17 |
| A | US 2014-0231273 A1 (MCCOLL et al.) 21 August 2014<br>See paragraphs [38]-[43] and figures 3A-3F. | 1-17 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 FEBRUARY 2018 (14.02.2018) | **14 FEBRUARY 2018 (14.02.2018)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

## INTERNATIONAL SEARCH REPORT

| International application No. |
|---|
| PCT/KR2017/005252 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

The invention of group 1: claims 1 to 10 pertain to a structure of a bio-measurement device.

The invention of group 2: claims 11 to 17 pertain to a bio-device for measuring and processing the concentration and/or blood sugar concentration of hematocrit and hemoglobin.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☒ No protest accompanied the payment of additional search fees.

Form PCT/ISA/210 (continuation of first sheet (2)) (January 2015)

## INTERNATIONAL SEARCH REPORT
Information on patent family members

International application No.

**PCT/KR2017/005252**

| Patent document cited in search report | Publication date | Patent family member | Publication date |
|---|---|---|---|
| US 2016-0320331 A1 | 03/11/2016 | US 2015-0377825 A1 | 31/12/2015 |
| WO 2007-128684 A1 | 15/11/2007 | EP 2021764 A1 | 11/02/2009 |
| | | IT UD20060111 A1 | 29/10/2007 |
| US 2013-0062221 A1 | 14/03/2013 | CA 2846887 A1 | 21/03/2013 |
| | | EP 2568281 A1 | 13/03/2013 |
| | | JP 2013-061336 A | 04/04/2013 |
| | | JP 5812957 B2 | 17/11/2015 |
| | | US 8603309 B2 | 10/12/2013 |
| | | WO 2013-039752 A1 | 21/03/2013 |
| US 2006-0051738 A1 | 09/03/2006 | US 2003-0068666 A1 | 10/04/2003 |
| | | US 6984307 B2 | 10/01/2006 |
| | | US 7758744 B2 | 20/07/2010 |
| US 2014-0231273 A1 | 21/08/2014 | AU 2012-300836 A1 | 02/05/2013 |
| | | AU 2012-300836 B2 | 12/02/2015 |
| | | EP 2751553 A1 | 09/07/2014 |
| | | HK 1199756 A1 | 17/07/2015 |
| | | US 9322800 B2 | 26/04/2016 |
| | | WO 2013-030369 A1 | 07/03/2013 |

Form PCT/ISA/210 (patent family annex) (January 2015)